(19) Europäisches Patentamt
European Patent Office
Office européen des brevets

(11) Veröffentlichungsnummer: **0 285 061**
**A2**

(12) # EUROPÄISCHE PATENTANMELDUNG

(21) Anmeldenummer: **88104969.6**

(22) Anmeldetag: **28.03.88**

(51) Int. Cl.⁴: **C07K 7/06**, C07K 7/30, A61K 37/02

(30) Priorität: **30.03.87 US 31632**

(43) Veröffentlichungstag der Anmeldung: **05.10.88 Patentblatt 88/40**

(84) Benannte Vertragsstaaten: **AT BE CH DE ES FR GB GR IT LI LU NL SE**

(71) Anmelder: **F. HOFFMANN-LA ROCHE & CO. Aktiengesellschaft**

**CH-4002 Basel(CH)**

(72) Erfinder: **Danho, Waleed**
**118 Bunker Hill Road**
**Wayne, N.J. 07470(US)**
Erfinder: **Madison, Vincent Stewart**
**12 Ronarm Drive**
**Mountain Lakes, N.J. 07046(US)**
Erfinder: **Triscari, Joseph**
**22 Evergreen Drive**
**North Caldwell, N.J. 07006(US)**

(74) Vertreter: **Lederer, Franz, Dr. et al**
**Patentanwält Dr. Franz Lederer Lucile Grahnstrasse 22**
**D-8000 München 80(DE)**

(54) **Cyclische Peptide und deren Herstellung.**

(57) Die Erfindung betrifft Peptide der allgemeinen Formeln

$$X\text{-}Tyr(SO_3H)\text{-}\underline{R\text{-}Gly\text{-}Trp\text{-}R^1\text{-}As}p\text{-}R^2\text{-}Y \qquad I$$

worin
X = CO-niederes Alkyl
R = Lys, Orn
$R^1$ = Met, Nle
$R^2$ = Phe, N-methyl-Phe
Y = $NH_2$, $NHCH_3$;

$$\underline{Tyr(SO_3H)\text{-}R\text{-}Glu}\text{-}Trp\text{-}R^1\text{-}R^2\text{-}N\text{-}methyl\text{-}Phe\text{-}Y \qquad II$$

worin
R = Met, Nle

EP 0 285 061 A2

$R^1$ = Met, Nle
$R^2$ = Thr($SO_3H$), Ser($SO_3H$), Hyp($SO_3H$)
Y = $NH_2$, $NHCH_3$
und

Tyr($SO_3H$)-R-Gly-Trp-Glu-$R^1$-N-methyl-Phe-Y    III

worin
R = Met, Nle
$R^1$ = Thr($SO_3H$), Ser($SO_3H$), Hyp($SO_3H$)
Y = $NH_2$, $NHCH_3$
sind, deren lineare Analoge und deren pharmazeutisch sinnvolle Salze mit Nahrungsaufnahme-unterdrückender oder -regulierender Wirkung. Die Verbindungen können durch Umsetzung der entsprechenden unsulfatierten Peptide mit einem Sulfatierungsreagens hergestellt werden und, falls gewünscht, durch Ueberführung der so erhaltenen Peptide in pharmazeutisch sinnvolle Salze und können zur Unterdrückung der Nahrungsaufnahme verwendet werden.

# Cyclische Peptide und deren Herstellung

In allen Ländern, die einen gewissen Wohlstand erreicht haben, stellt Fettleibigkeit einen der Faktoren steigender Gesundheitsfürsorgekosten dar. So liegen beispielsweise annähernd 34 Millionen Amerikaner über ihrem Idealgewicht. Für wenigstens 20% davon ist gemäss den Entschlüssen einer kürzlich abgehaltenen Konferenz des NIH eine Behandlung ratsam [National Institutes of Health Consensus Panel Report, February 13, (1985); siehe auch: Science, 227, 1019-1020 (1985)].

Bei solchen Individuen stellt Fettleibigkeit einen zusätzlichen Faktor für vermehrtes Auftreten von Herzkreislauferkrankungen, Hochdruck, Hypercholesterinämie, insulinunabhängiger Diabetes und Uterus-, Brust-, Gallenblasen-, Dickdarm-, Enddarm-und Prostatakrebs dar. Zusätzlich dazu verkürzt Fettleibigkeit die Lebensdauer, sodass in ausserordentlichen oder gar pathologischen Fällen von Fettleibigkeit die Mortalitätsrate 1200 Prozent über dem Normalwert liegt.

Die Verminderung des Gewichts wird bei Patienten, die an NIDD, Hochdruck, Hypercholesterolämie, Herzkranzgefässerkrankungen, Gicht und Osteoarthritis leiden, als ersten Therapieschritt empfohlen. Jedoch gibt es relativ wenige Therapeutika die der Arzt benützen kann, um Abmagerung zu erreichen. Pharmazeutika, die gewöhnlich zur Unterstützung einer Diät verwendet werden, sind im Bereich einer Kurzzeittherapie wirkungsvoll. Auf grund der Ausbildung von Toleranz, ihrer ZNS-Aktivität und unerwünschter Nebeneffekte sind sie für eine Langzeittherapie nicht geeignet. So kehren etwa 95% der Patienten, die erfolgreich Gewicht verloren haben, innerhalb von 12-84 Monaten zu ihrem anfänglichen Körpergewicht zurück. Von einem Mittel, welches die Nahrungsaufnahme durch Vortäuschung eines dem eigenen Körper ähnlichen peripheren Sättigungssignals reduzierte, würde man erwarten, dass es erfolgreicher bei der Behandlung chronischer Zustände sein, weniger unerwünschte Nebeneffekte haben und frei von ZNS-Aktivität sein sollte.

Cholecystokinin (CCK) ist ein Polypeptidhormon, das erstmals als Peptid mit einer Länge von 33 Aminosäuren aus dem Gastrointestinaltrakt des Schweines isoliert wurde [Mutt et al., Biochem. J. (Proced. Biochem. Soc.), 1971, 125, 57p-58p; Mutt et al., Clin. Endocrinol., Supplement, 1976, 5, 175s-183s]. Von peripher verabreichtem CCK konnte gezeigt werden, dass es zu Sättigung in der Ratte, dem Schaf und dem Affen führt (Jorpes et al., Acta. Chem. Scand., 1964, 18, 2408-2410; Della-Fera et al., Science, 1979, 206, 471-473; Gibbs et al., 1973, J. Comp. and Physiol. Psychol., 84, 488-495). Infusionen von CCK-8, dem Octapeptidanalogen des CCK, führten bei Mageren wie Fettleibigen zu verminderter Nahrungsaufnahme (J. Smith, Int. J. of Obesity, 1984, 8, Suppl. 1, 35-38). Es ist heutzutage allgemein anerkannt, dass CCK sättigende Wirkung hat und deshalb zur Verminderung oder Unterdrückung der Nahrungsaufnahme beim Menschen eingesetzt werden könnte.

Das Polypeptidhormon CCK-33 hat die Aminosäuresequenz:

```
1                   5                   10
Lys-Ala-Pro-Ser-Gly-Arg-Val-Ser-Met-Ile-
                    15                  20
Lys-Asn-Leu-Gln-Ser-Leu-Asp-Pro-Ser-His-
                    25                            30
Arg-Ile-Ser-Asp-Arg-Asp-Tyr(SO3H)-Met-Gly-Trp-
           33
Met-Asp-Phe-NH2.
```

Auch von CCK-Fragmenten, wie beispielsweise CCK-8 und CCK-7, konnte eine sättigungsinduzierende Wirkung gezeigt werden. CCK-8 hat die Aminosäuresequenz:

```
26  27             28  29  30  31  32  33
Asp-Tyr(SO3H)-Met-Gly-Trp-Met-Asp-Phe-NH2.
```

CCK-7 verfügt über eine Aminosäure weniger wie CCK-8, das heisst, dass es mit CCK-8, dem Asp an Stelle 26 fehlt, identisch ist.

Die bekannten Peptide haben eine lineare Konfiguration.

Im allgemeinen sind lineare Peptide sehr flexible Moleküle und es fehlt ihnen eine genau definierte Konformation. Jede Aminosäure in einem linearen Peptid ist dem sie umgebenden Milieu ausgesetzt, was zu einem Peptid führt, das enzymatischem und chemischem Abbau zugänglicher ist als ein solches mit einer wohldefinierten Konformation.

Es ist bekannt, dass die linearen Formen von CCK-33, CKK-8, und dessen verschiedenen Analoga, wie auch von CCK-7 sättigungsinduzierende Wirkungen haben, wobei aber die Wirkungsdauer dieser Peptide kurz ist. Beispielsweise wird lineares CCK-8 unter Einwirkung von menschlichem Magensaft sehr schnell abgebaut. Dadurch wird eine wirkungsvolle orale Verabreichung sättigungsinduzierender Verbindungen ausgeschlossen. Das Auffinden von sättigungsinduzierenden Verbindungen mit länger anhaltender Wirkung sowie eine wirkungs volle Weise der oralen Verabreichung ist deshalb von Interesse.

Unter einem cyclischen Peptid versteht man ein Peptid, in dem der beta-oder gamma-Carboxyterminus einer Aminosäure in der Peptidkette mit dem alpha-, delta-, oder epsilon-Aminoterminus einer anderen Aminosäure in der Peptidkette durch Ausbildung einer Amidbindung verbunden ist. Die Bindung zwischen den beiden Aminosäuren in der Kette führt zu einer Ringstruktur.

Die biologischen Eigenschaften cyclischer Peptide weichen erheblich von denen ihrer linearen Analogen ab. Cyclische Peptide sind starrer und besitzen wohldefinierte Strukturen und im Inneren liegende Aminosäurereste, die vom umgebenden Milieu abgeschirmt sind. Diese Unterschiede zeigen sich in den biologischen Eigenschaften der Peptide. Die Wirkung der cyclischen Peptide wird länger anhalten, da die kompakte Struktur sie chemischem wie enzymatischem Abbau weniger zugänglich macht. Auf Grund veränderter Gewebsverteilung, verursacht durch die abgeschirmten inneren Aminosäurereste, wird die biologische Verfügbarkeit der cyclischen Peptide verbessert sein. Auf Grund der wohldefinierten Struktur werden die cyclischen Peptide eine grössere Spezifität für den Zielrezeptor zeigen und dadurch die Wahrscheinlichkeit unerwünschter Nebenwirkungen vermindern. Im Gegensatz dazu gibt es für lineare Peptide im allgemeinen zentrale wie periphere Rezeptoren und es besteht für ein gegebenes Peptid mit dem Rezeptor für ein anderes Peptid eine erhebliche Kreuzreaktivität.

Die vorliegende Erfindung betrifft lineare Peptide spezifischer Sequenzen, cyclische Analoge dieser Peptide und ihre pharmazeutisch sinnvollen Salze. Die Erfindung betrifft im weiteren Methoden zur Unterdrückung der Nahrungsaufnahme bei Tieren, die dadurch gekennzeichnet sind, dass man besagten Tieren eine so grosse Menge der erfindungsgemässen Peptide oder ihrer pharmazeutisch sinnvollen Salze verabreicht, dass sie ausreicht, um die Nahrungsaufnahme wirkungsvoll zu unterdrücken.

In der vorliegenden Erfindung ist ein cyclisches Peptid dadurch definiert, dass es sich dabei um ein Peptid handelt worin der beta-oder gamma-Carboxyterminus einer Aminosäure in der Peptidkette an den alpha-, delta-oder epsilon-Aminoterminus einer anderen Aminosäure in dieser Peptidkette gebunden ist. Falls nicht anders angegeben, werden die folgenden Konfigurationen für die erfindungsgemässen Peptide verwendet.

| Aminosäure in der Kette | Funktionelle Gruppe, über die das cyclische Peptid gebildet wird | |
|---|---|---|
| Lys | ε amino | (ε=epsilon) |
| Orn | Δ amino | (Δ=delta) |
| Tyr($SO_3H$)) | α amino | (α=alpha) |
| Asp | ß carboxy | (ß=beta) |
| Glu | γ carboxy | (γ=gamma) |

Die erfindungsgemässen cyclischen Peptide werden dadurch hergestellt, dass man die funktionellen Gruppen der Aminosäuren in der Kette, an denen Cyclisierung unerwünscht ist, schützt und die Aminosäuren, über die Cyclisierung erfolgen soll, ungeschützt lässt. Für die Synthese an einer festen Phase sind die funktionellen Gruppen der verschiedenen Aminosäuren in üblicher Weise mit passenden Schutzgruppen, die eine chemische Reaktion dort solange verhindern, bis die Schutzgruppen schliesslich entfernt werden, geschützt. Jede Aminosäure in der Kette kann durch jede für die entsprechende Aminosäure in der Synthese in flüssiger Phase im allgemeinen verwendete Schutzgruppe geschützt werden. Bindung zwischen den ungeschützten Aminosäuren in der Kette führt dann zu den erfindungsgemässen cyclischen Peptiden.

In der vorliegenden Beschreibung und den Patentan sprüchen werden die folgenden Abkürzungen oder Symbole verwendet:

| Symbol | Bedeutung |
|---|---|
| AA | Aminosäure |
| Ac | Acetyl |
| Orn | Ornithin |
| 2-chloro-Z | 2-Chloro-benzyloxycarbonyl |
| Z | Benzyloxycarbonyl |
| DMF | Dimethylformamid |
| Boc | tert.-Butyloxycarbonyl |
| TFA | Trifluoressigsäure |
| $CH_3CN$ | Acetonitril |
| Phe-$NH_2$ | 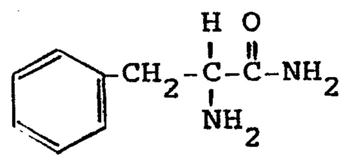 |
| N-methyl-Phe-$NH_2$ | 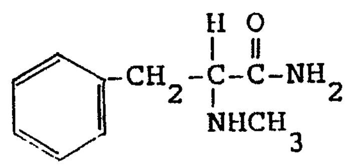 |
| Phe-$NHCH_3$ | 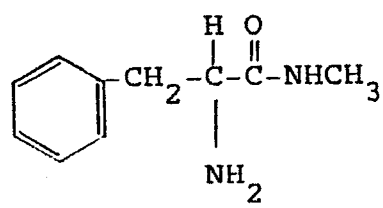 |
| N-methyl-Phe-$NHCH_3$ | 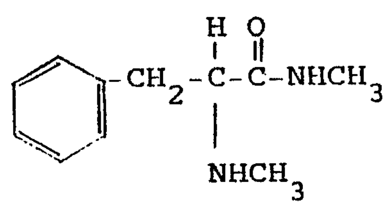 |

Die Aminosäuren werden mit den üblichen Abkürzungen in Form von jeweils 3 Buchstaben bezeichnet und falls nicht anders angegeben handelt es sich dabei jeweils um das L-Isomere.

Die Erfindung betrifft cyclische Peptide der Formeln:

$$\text{X-Tyr(SO}_3\text{H)-}\underline{\text{R-Gly-Trp-R}^1\text{-As}}\text{p-R}^2\text{-Y} \qquad \text{(I)}$$

worin

X = CO-niederes Alkyl

R = Lys, Orn
$R^1$ = Met, Nle
$R^2$ = Phe, N-methyl-Phe
Y = $NH_2$, $NHCH_3$.

Besonders bevorzugt sind Peptide der Formeln:

$CH_3$-CO-Tyr($SO_3H$)-Lys-Gly-Trp-Met-Asp-N-methyl-Phe-$NH_2$

$CH_3$-CO-Tyr($SO_3H$)-Lys-Gly-Trp-Met-Asp-Phe-$NH_2$

$CH_3$-CO-Tyr($SO_3H$)-Lys-Gly-Trp-Nle-Asp-N-methyl-Phe-$NH_2$

$CH_3$-CO-Tyr($SO_3H$)-Orn-Gly-Trp-Met-Asp-N-methyl-Phe-$NH_2$

Die Erfindung betrifft auch Peptide der Formel:

Tyr($SO_3H$)-R-Glu-Trp-$R^1$-$R^2$-N-methyl-Phe-Y (II)

worin
R = Met, Nle
$R^1$ = Met, Nle
$R^2$ = Thr($SO_3H$), Ser($SO_3H$), Hyp($SO_3H$)
Y = $NH_2$, $NHCH_3$.

Besonders bevorzugt ist ein Peptid der Formel:

Tyr($SO_3H$)-Met-Glu-Trp-Met-Thr($SO_3H$)-N-methyl-Phe-$NH_2$

Die Erfindung bezieht sich auch auf Peptide der Formel:

Tyr($SO_3H$)-R-Gly-Trp-Glu-$R^1$-N-methyl-Phe-Y (III)

worin
R = Met, Nle
$R^1$ = Thr($SO_3H$), Ser($SO_3H$), Hyp($SO_3H$)
Y = $NH_2$, $NHCH_3$

Besonders bevorzugt ist ein Peptid der Formel:

Tyr($SO_3H$)-Met-Gly-Trp-Glu-Thr($SO_3H$)-N-methyl-Phe-$NH_2$

Im Hinblick auf Peptide der Formel I, worin R = Lys, ist es der ε-Aminoterminus des Lys der an den β-Carboxyterminus des Asp zur Bildung des cyclischen Peptids gebunden ist. Ist in Formel I R = Orn, dann ist es der Δ-Aminoterminus des Orn, der an den β-Carboxyterminus des Asp zur Bildung des cyclischen Peptids gebunden ist.

Bei Verbindungen der Formel II ist es der α-Aminoterminus des Tyr($SO_3H$), der an den γ-Carboxyterminus des Glu gebunden wird und zur Bildung des cyclischen Peptids führt.

Bei Verbindungen der Formel III ist es der α-Aminoterminus des Tyr($SO_3H$), der an den γ-Carboxyterminus des Glu gebunden wird und zur Bildung des cyclischen Peptids führt.

Die Erfindung betrifft auch lineare Peptide der Formeln

X-Tyr($SO_3H$)-R-Gly-Trp-$R^1$-Asp-$R^2$-Y (IV)

worin
X = CO-niederes Alkyl
R = Lys, Orn
$R^1$ = Met, Nle
$R^2$ = Phe, N-methyl-Phe
Y = $NH_2$, $NHCH_3$,

Tyr($SO_3H$)-R-Glu-Trp-$R^1$-$R^2$-N-methyl-Phe-Y (V)

worin
R = Met, Nle
$R^1$ = Met, Nle
$R^2$ = Thr($SO_3H$), Ser ($SO_3H$), Hyp($SO_3H$)
Y = $NH_2$, $NHCH_3$

Tyr($SO_3H$)-R-Gly-Trp-Glu-$R^1$-N-methyl-Phe-Y (VI)

worin
R = Met, Nle
$R^1$ = Thr($SO_3H$), Ser($SO_3H$), Hyp($SO_3H$)
Y = $NH_2$, $NHCH_3$.

Die erfindungsgemässen Peptide verfügen über sättigungsinduzierende Wirkung bei Tieren und können zur Unterdrückung der Nahrungsaufnahme verwendet werden. Sie können in einem Programm zur Kontrolle und Verminderung des Körpergewichts von Tieren verwendet werden.

Die Erfindung betrifft cyclische Peptide der Aminosäuresequenzen gemäss den Formeln I, II, III, deren pharmazeutisch sinnvolle Salze und Zusammensetzungen, die diese Peptide enthalten.

Die Erfindung betrifft ferner lineare Peptide der Aminosäuresequenzen gemäss den Formeln IV, V und VI und deren pharmazeutisch sinnvolle Salze.

Die Erfindung betrifft auch eine Methode zur Unterdrückung der Nahrungsaufnahme bei Tieren, die dadurch charakterisiert ist, dass man besagten Tieren eine zur Untedrückung der Nahrungsaufnahme ausreichende Menge an erfindungsgemässen Peptiden verabreicht.

Der hier verwendete Ausdruck "Menge zur Unterdrückung der Nahrungsaufnahme" bezieht sich auf die Menge an Peptid (auf Gewichtsbasis), die pro kg Körpergewicht des Tieres zum Unterdrücken der Nahrungsaufnahme verabreicht werden muss. Die Berechnung der entsprechenden Mengen bezüglich der Verabreichungsmethode, des entsprechenden Tieres und des Körpergewichtes des Tieres gehört zum Stand der Technik. Der Stand der Technik bezüglich der Verwendung von CCK-8 als Appetitzügler wird durch die Referenzen, die in Morley, J.E., "Minireview-The Ascent of Cholecystokinin (CCK)-From Gut to Brain", Life Sciences (1982), 30, 479-493, auf den Seiten 485-488 zusammengefasst sind, veranschaulicht.

Das Verfahren zur Synthese der erfindungsgemässen Peptide setzt sich folgendermassen zusammen:

a) Herstellung eines entsprechenden blockierten, linearen, an ein Festphasenträgermaterial gebundenen Peptids.

b) Entfernung des linearen Peptids vom Trägermaterial und Reinigung durch HPLC.

c) Umsetzung des linearen Peptids mit einem Cyclisierungsmittel zur Synthese des cyclischen Peptids durch Ausbildung einer Amidbindung und Reinigung durch HPLC.

d) Sulfatierung des cyclischen Peptids und Reinigung durch HPLC.

a) Herstellung des blockierten, linearen, an ein Festphasenträgermaterial gebundenen Peptids:

Das Peptid kann mittels Synthese an festen Phasen nach der von Merrifield [J. Am. Chem. Soc., 85, 2149-2154 (1963)] im allgemeinen beschriebenen Methode oder durch Verwendung einer anderen, äquivalenten, im Stand der Technik beschriebenen, chemischen Synthesemethode hergestellt werden. Die Synthese an festen Phasen beginnt am C-terminalen Ende des zu synthetisierenden Peptids durch Kopplung einer geschützten α-Aminosäure durch Amidbindung an ein passendes Trägermaterial, z.B. Benzhydrylamin (BHA)-oder Methylbenzhydrylaminträgermaterial (MBHA). BHA-und MBHA-Trägermaterialien sind käuflich erhältlich und werden im allgemeinen verwendet, wenn das gewünschte zu synthetisierende Peptid über eine unsubstituierte Amidgruppe am Carboxyterminus verfügt.

Alle für die hier beschriebenen Synthesen verwendeten Lösungsmittel, z.B. Methylenchlorid ($CH_2Cl_2$), 2-Propanol und Dimethylformamid (DMF), hatten den Reinheitsgrad "Distilled in Glass" von Burdick & Jackson und wurden ohne zusätzliche Destillation verwendet. Trifluoressigsäure (TFA), Diisopropylethylamin (DIPEA) und Dicyclohexylcarbodiimid (DCC) wurden von Chemical Cyanamid Corporation bezogen und hatten den Reinheitsgrad "sequential". Ethandithiol (EDT) wurde von Sigma Chemical Co. bezogen und ohne weitere Reinigung verwendet. Alle geschützten Aminosäuren wurden von Bachem bezogen und hatten, ausser dass es anders angegeben ist, die L-Konfiguration.

In der Festphasensynthese werden die reaktiven Seitengruppen der verschiedenen Aminosäuren typischerweise mit geeigneten Schutzgruppen, die eine chemische Reaktion an diesen Stellen verhindern bis die Schutzgruppen entfernt werden, geschützt. Spezifische Schutzgruppen sind im Hinblock auf die Festphasensynthese offenbart, aber es sollte angemerkt werden, dass jede Aminosäure durch jede üblicherweise für die entsprechende Aminosäure in der Flüssigphasensynthese verwendete Schutzgruppe geschützt werden kann. Zu solchen Schutzgruppen gehören beispielsweise die üblichen Schutzgruppen für Carboxylgruppen aus der Gruppe der Ester wie beispielsweise Arylester, im besonderen Phenyl-oder durch niederes Alkyl, Halogen, Nitro, Thio oder niederes Alkyl(1-7 C-Atome)thio substituierte Phenylester. Alle α-Aminogruppen wurden mit tert.-Butyloxycarbonyl-(Boc)-Funktionen blockiert. Seitengruppen wurden wie folgt substituiert: Asp, Glu, Thr mit Benzyl; Trp mit Formyl und Tyr mit 2,6-Dichlorobenzyl; Lys mit 2-Chloro-Z oder mit Z. Die Reinheit dieser Verbindungen wurde durch Dünnschichtchromatographie (TLC) und optische Rotation bestätigt. Das BHA-Trägermaterial stellt ein Copolymer aus Styrol und 1% Divinylbenzol in Form von Kügelchen (Porengrösse 200-400) dar, das von Beckmann Instruments erhalten wurde. Der gesamte Stickstoffgehalt betrug 0,654 meq/g.

Die folgenden Geräte wurden verwendet: Dünnnschichtchromatographie (TLC) wurde auf vorbeschichteten, gebackenen Glass-Silicagelplatten Typ 60 F254 (Merck) mittels geeigneter Lösungsmittelsysteme durchgeführt. Der Nachweis der Flecken erfolgte durch UV-Fluoreszenzquenching (254 nm Absorption), Markierung mit Jod oder mittels Ninhydrin (für primäre und sekundäre Amine).

Zur Aminosäureanalyse wurden die Peptide in 6N Salzsäure, die Phenol enthielt, in evakuierten "Reacti-Therm"-Hydrolysegläsern (Wheaton Scientific Company, Milville, N.J., USA) in 24 Stunden bei 115°C hydrolysiert. Die Analysen wurden auf einem Beckman 121M Aminosäureanalysator durchgeführt.

Hochdruckflüssigkeitschromatographie (HPLC) wurde auf einem Labordatenkontrollapparat (LDC), der aus einer constametrischen I-Pumpe, einer constametrischen III-Pumpe, einem "Gradient Master"-Lösungsmittelprogrammierer und Mischer und einem Spektrophotometermonitor III mit variabler UV-Wellenlängendetektion durchgeführt. Analytische HPLC wurde auf Micro-Bondapack-$C_{18}$-Umkehrphasensäulen (0,4 × 25 cm) von Waters durchgeführt. Präparative HPLC wurde auf einer Partisil-M20-10/50-ODS-3-Säule (2,5 × 50 cm) von Whatman oder einer Micro-Bondapack-$C_{18}$-Säule (2,3 × 30 cm) durchgeführt. In beiden Fällen wurde eine Co:Pell-ODS-("Pellicular packing")-Vorsäule von Whatman verwendet.

Die Peptide wurden schrittweise an einer festen Phase mittels einer Peptidsynthesemaschine, Modell Vega 250, aufgebaut. Der Ablauf der chemischen Reaktionen wurde über einen Mikroprozessor, Modell 300 von Vega Biochemicals, mit Handbetrieb bei den Schritten 16 und 20, kontrolliert.

5 g Boc-N-methyl-Phe (17 mMol) wurde auf das Benzhydrylaminträgermaterial (5 g) mittels 1,8 g DCC (9 mMol) bei 0°C gekoppelt. Eine Ladung von 0.2mMol/g Trägermaterial wurde mittels Aminosäureanalyse bestimmt. Jede freie Aminogruppe wurde mit jeweils 6 Aequivalenten Essigsäureanhydrid und Pyridin blockiert.

Die Synthese wurde mit dem Trägermaterial begonnen.

## Das Protokoll für einen typischen Synthesezyklus:

| Schritt | Reagens | Zeit |
|---|---|---|
| 1 | 1% EDT/$CH_2Cl_2$ | 1x30 sek. |
| 2 | 50% TFA/$CH_2Cl_2$ 1% EDT | 1x1 min. |
| 3 | Wiederholung Schritt 1 | |
| 4 | 50% TFA/$CH_2Cl_2$ 1% EDT | 1x15 min. |
| 5 | $CH_2Cl_2$ | 1x30 sek. |
| 6 | 2-Propanol | 1x30 sek. |
| 7-8 | Wiederholung der Schritte 5 und 6 | |
| 9 | $CH_2Cl_2$ | 2x30 sek. |
| 10 | 8% DIPEA | 2x2 min. |
| 11-15 | Wiederholung der Schritte 5-9 | |
| 16 | 5 equiv. Boc-AA-Anhydrid | 1x30 min. |
| 17 | 1% DIPEA | 1x5 min. |
| 18-19 | Wiederholung der Schritte 6 und 9 | |
| 20-21 | Wiederholung der Schritte 16 und 17, falls der Test nach Kaiser positiv ist | |
| 22 | 2-Propanol | 1x30 sek. |
| 23-24 | Wiederholung der Schritte 5 und 6 | |
| 25 | $CH_2Cl_2$ | 1x30 sek. |
| 26 | DMF | 2x30 sek. |
| 27 | $CH_2Cl_2$ | 3x30 sek. |

Die Lösungsmittel für alle Wasch-und Kopplungsschritte wurden auf Volumina von 10-20 ml/g Trägermaterial bezogen. Die Kopplungen wurden in Form der symmetrischen Anhydride der Boc-Aminosäuren in $CH_2Cl_2$ mit 10 Aequivalenten Boc-Aminosäure und 5 Aequivalenten DCC in 15 minuten bei 0°C durchgeführt.

Die Kopplungsreaktionen wurden mit dem "Kaiser"-Ninhydrintest überwacht, um zu bestimmen ob vollständige Kopplung nach Schritt 19 erfolgt war [Kaiser, E. et al., Anal. Biochem., 34, 595-598, (1970)]. Die Zeiten für einen vollständigen Zyklus betrugen 54-160 Minuten pro Aminosäure.

b) Entfernung des linearen Peptids vom Trägermaterial und Reinigung durch präparativen HPLC:

Die fertig synthetisierten, an die Trägermaterialien gebundenen Peptide wurden im Hochvakuum getrocknet. Als Deblockierungs-und Spaltungsbedingung wurden die von Tam et al. [Tetrahedron Letters, 23, 4435-4438, (1982)] modifizierten verwendet, die im allgemeinen für CCK-Analoge nochmals verbessert wurden. Die an das Trägermaterial gebundenen Peptide wurden in einem Teflon-HF-Apparat (Peninsula) mit HF, Dimethylsulfid und p-Kresol im Verhältnis 5:13:2 in ml 1 Stunde lang bei 0°C behandelt. Nach Einengung auf ein kleines Volumen wurde 18 ml wasserfreier HF für eine zweite Umsetzung von 1,5 Stunden Dauer bei 0°C in das Reaktionsgefäss destilliert. Nach kräftiger Einengung wurde das trockene Trägermaterial mit jeweils 3 Volumen $Et_2O$ und EtOAc gewaschen, viermal mit jeweils 15 ml 30%iger

Essigsäure behandelt und filtriert. Gefriertrocknung des wässrigen Filtrats ergab das rohe lineare Peptid.

Die präparative Reinigung wurde durch HPLC mit dem so erhaltenen Peptid auf einer Mikrobondapack-$C_{18}$-Säule (2,3 × 30 cm) oder einer ODS-3-Säule (2,5 × 50 cm) von Whatman durchgeführt. Die Peptide wurden in einem möglichst kleinen Volumen 50%iger AcOH aufgetragen und mit einem flachen Gradienten (4 Stunden) von 5-65% 0,022% TFA/$CH_3CN$ bei einer Flussrate von 8,0 ml/Min. eluiert. Die Fraktionen wurden in Intervallen von 3 Minuten gesammelt und Einschnitte erfolgten nach Prüfung durch analytische HPLC. Fraktionen mit einer Reinheit über 97% wurden gesammelt und gefriergetrocknet.

Die Reinheit der Peptide wurde durch HPLC überprüft. Sie betrug in allen Fällen 99%. Aminosäureanalysen der einzelnen Peptide bestätigten die erwarteten Werte. UV, IR, und MS wurden auch mit den Analogen durchgeführt und bestätigten die chemische Unversehrtheit der Peptide.

c) Umsetzung der linearen Peptide mit einem Cyclisierungsmittel und Reinigung der cyclischen Peptide durch HPLC:

Die linearen Peptide wurden in DMF gelöst und mit 3N HCl/Dioxan zur Protonierung der freien Aminogruppe in Form des Hydrochloridsalzes umgesetzt. Dieses wurde mit Diphenylphosphenylazid umgesetzt und 1 Stunde lang bei -20°C aktiviert. Anschliessend wurde die Reaktionsmischung mit DMF verdünnt (15 Volumina) und N-Methylmorpholin wurde bis zu einem pH von 7,5 zugegeben. Die Cyclisierungsreaktion wurde in 2 Tagen bei 5°C durchgeführt. Während dieser Zeit wurde der pH gemessen und im Neutralen (pH 7,4) durch Zugabe von N-Methylmorpholin gehalten. Der "pH" in einem organischen Lösungsmittel wird durch Auftragen eines Aliquots der Lösung auf angefeuchtetes pH-Papier, das einen - schmalen pH-Bereich umfasst, bestimmt.

Der Cyclisierungsverlauf wurde durch HPLC-Analyse eines Aliquots der Reaktionsmischung verfolgt. Im allgemeinen ist das lineare Ausgangspeptid innerhalb von 1-2 Tagen in das cyclische Monomere oder die polymeren Formen des linearen Peptids umgewandelt.

Die so erhaltene rohe Mischung der cyclischen Peptide wurde durch präparative HPLC auf einer Mikro-Bondapack $C_{18}$-Säule (2,3 × 30 cm) gereinigt. Die Peptide wurden in einem möglichst kleinen Volumen Essigsäure aufgetragen und mit einem flachen Gradienten (4 Stunden) von 5-65% 0,022% TFA/$CH_3CN$ bei einer Flussrate von 8,0 ml/Min. eluiert. Fraktionen wurden in Intervallen von 3 Minuten gesammelt und Einschnitte erfolgten nach Prüfung durch analytische HPLC. Die Reinheit der cyclischen Peptide wurde durch analytische HPLC, Aminosäureanalyse und MS bestimmt.

d) Sulfatierung der cyclischen Peptide und Reinigung durch HPLC:

Die Schwefelsäureestergruppe enthaltenden Peptide wurden durch doppelte Sulfatierung der Hydroxylgruppen (Tyrosin, Serin, Threonin oder Hydroxyprolin) mit Pyridinacetylschwefelsäure hergestellt. Eine typische Sulfatierung wurde folgendermassen durchgeführt: 60-240 mg Pyridinacetylsulfat (PAS) wurden in 5 ml Pyridin gelöst und 10 Minuten bei 60°C vermischt. Das N-Acetyl-CCK-8 Analoge (10 mg) wird in 5 ml Pyridin gelöst und PAS dazugegeben. Nach Erhitzen und 45-60-minütigem Mischen bei 60°C wird mit 2 Volumina 0,05 M Ammoniumbicarbonat neutralisiert, lyophilisiert und mittels HPLC gereinigt.

Die sulfatierten Peptide wurden durch präparative Umkehrphasen-HPLC an $C_{18}$-10 µ(ES Industries; 1,25 × 30 cm) Säulen mit einem 2-stündigen Gradienten (10-40%) von Acetonitril in 0,05 M Ammoniumbicarbonat, bei einer Flussrate von 5 ml/Min. und Nachweis bei 240 nm, gereinigt. Die zu sammelnden Fraktionen und die Reinheit der Peptide wurden durch analytische HPLC an Bondapack $C_{18}$-10 µSäulen (0,3 × 30 cm) von Waters mit einem Gradienten von Acetonitril in Ammoniumbicarbonat, bei einer Flussrate von 2 ml und Nachweis bei 215 nm, bestimmt.

Die Reinheit der sulfatierten Peptide wurde durch analytische HPLC, Aminosäureanalyse, UV, IR, MS und NMR bestimmt.

Die folgenden Beispiele veranschaulichen im Detail die Herstellung der erfindungsgemässen, appetitregulierenden Peptide mittels der oben beschriebenen Verfahren. Die Peptide wurden, solange es nicht anders erwähnt ist, charakterisiert und ihre Reinheit wurde durch Aminosäureanalyse, analytische HPLC, UV, IR und MS bestimmt.

<u>Beispiel 1</u>

Herstellung von

Ac-Tyr(SO$_3$H)-Lys-Gly-Trp-Met-Asp-N-methyl-Phe-$NH_2$

Boc-N-methyl-Phe (5 g, 17,8 mMol) wurde in einer Mischung aus 50 ml Methylenchlorid und 50 ml Dimethylformamid gelöst, auf 0°C abgekühlt und Dicyclohexylarbodiimid (1,8 g, 9 mMol) wurde unter Rühren dazugegeben und die Mischung 60 Minuten lang bei 0°C weitergerührt.

Getrennt davon wurde 5 g Benzhydrylamin-Trägermaterial (0,56 mMol N/g) (quervernetztes Copolymer aus Styrol und 1% Divinylbenzol) mit 10%igem Diisopropylethylamin in Methylenchlorid 30 Minuten lang geswaschen, filtriert und anschliessend mit Methylenchlorid, Dimethylformamid und Methylenchlorid gewaschen. Die abgekühlte Mischung (siehe oben) wurde zum Trägermaterial gegeben und 24 Stunden lang bei Raumtemperatur gerührt. Das Trägermaterial wurde abfiltriert, mit Methylenchlorid, Dimethylformamid, Isopropanol, Methylenchlorid, Dimethylformamid, Isopropanol, und Methylenchlorid gewaschen und im Hochvakuum getrocknet.

Aminosäureanalyse zeigte, dass das Trägermaterial 0,2 mMol N-Methylphenylalanin pro Gramm Trägermaterial enthielt. Nichtreagierte Aminogruppen wurden durch 60-minütiges Schütteln des Trägermaterials mit 5 ml Essigsäureanhydrid und 5 ml Diisopropylethylamin in Methylenchlorid blockiert. Das Trägermaterial wurde abfiltriert und mit Methylenchlorid, Isopropanol, Dimethylformamid und Methylenchlorid gewaschen. 4,8 g (0,96 mMol) Boc-N-Methylphenylalanin-Trägermaterial wurde nach dem oben beschriebenen Verfahren einer Abfolge von Festphasensyntheseschritten ausgesetzt. Alle Kopplungsschritte wurden wie beschrieben mit den symmetrischen Anhydriden der Boc-Aminosäuren durchgeführt. Bei Schritt 16 und 20 wurden die aktivierten Aminosäuren zugegeben. Die entsprechenden Reaktionszeiten waren die folgenden: 6 Zyklen wurden mit Boc-asparaginsüure-β-benzylester (1,6 g, 5 mMol, 60 Minuten; 1,6 g, 5 mMol, 60 Minuten), Boc-methionin (1,25 g, 5 mMol, 30 Minuten; 1,25 g, 30 Minuten, 5 mMol), Boc-N$^1$-formyltryptophan (1,7 g, 5 mMol, 30 Minuten; 1,7 g, 5 mMol, 30 Minuten), Boc-glycin (900 mg, 5 mMol, 30 Minuten; 900 mg, 5 mMol, 30 Minuten), Boc-ε-2-chlor-Z-lysin (2,1 g, 5 mMol, 30 Minuten; 2, 1 g, 5 mMol, 30 Minuten) und Boc-2,6-dichlorbenzyl-tyrosin (2,2 g, 5 mMol, 30 Minuten; 2,2 g, 5 mMol, 30 Minuten) durchgeführt.

Abspalten der Boc-Schutzgruppen und Acetylierung mit 20 ml Essigsäureanhydrid und 20 ml Pyridin in Methylenchlorid in 60 Minuten ergab 5,2 g des acetylierten Heptapeptid-Trägermaterials

1,9 g des Peptid-Trägermaterials wurden durch Umsetzen mit 25 ml HF, der Dimethylsulfid (2 ml), Anisol (1 ml) und Dithioethan (0,7 ml) enthielt, 1 Stunde lang bei 0°C gespalten. Nach starker Einengung wurde das Trägermaterial mit 2 Volumina Ethylacetat gewaschen, dann viermal mit jeweils 15 ml 30%iger Essigsäure behandelt, filtriert und lysophilisiert, was 300 mg des rohen Peptids ergab.

150 mg des rohen Peptids wurden durch präparative HPLC an einer Mikor-Bondapack $C_{18}$-Säule (2,3 × 30 cm) gereinigt. Das Peptid wurde mit einem linearen Gradienten von 5-65% 0,022% TFA/$CH_3CN$ bei einer Flussrate von 8 ml/Min. gereinigt. Der Nachweise erfolgte bei 280 nm. Der Hauptpeak wurde gesammelt und lyophilisiert, was 20 mg (11%) Ac-Tyr-Lys-Gly-Trp-Met-Asp-N-methyl-Phe-$NH_2$ ergab. Dieses Material war nach HPLC-Kriterien homogen und ergab korrekte Aminosäureanalyse-und MS-Daten. 19 mg (0,02 mMol) des linearen Peptids wurden in 1 ml DMF gelöst und 0,1 ml 3M HCl/Dioxan wurden zugegeben. Die Reaktionsmischung wurde bis zur Trockene eingeengt und in 1 ml DMF wieder aufgenommen. Diphenylphosphorylazid (0,13 ml; 0,005 mMol) wurden, nachdem die Mischung auf -20°C abgekühlt worden war, zugegeben und die Mischung wurde 1 Stunde lang bei -20°C rühren gelassen. Danach wurde die Reaktionsmischung mit 15 ml DMF verdünnt und der pH mit N-Methylmorpholin auf 7,5 eingestellt (gemessen mit angefeuchteten pH Stäbchen). Die Reaktionsmischung wurde auf 5°C aufgewärmt und man liess sie 2 Tage bei dieser Temperatur und einem pH von 7,4 stehen. Der pH wurde durch Zugabe geringer Mengen N-Methylmorpholins konstant gehalten.

Die Reaktionsmischung wurde im Vakuum konzentriert und der Rückstand in 1 ml Essigsäure aufgelöst und auf eine Mikro-Bondapack $C_{18}$ Säule (2,3 × 30 cm) gegeben. Die Säule wurde mit einem flachen Gradienten (4 Stunden) von 5-65% 0,022% TFA/$CH_3CN$ bei einer Flussrate von 8 ml/Min. eluiert. Der Nachweis erfolgte bei 280 nm. Fraktionen wurden in Intervallen von 3 Minuten gesammelt und Einschnitte erfolgten nach Untersuchung durch analytische HPLC. Die das cyclische Peptid enthaltenden Fraktionen wurden gesammelt und lyophilisiert, was 8 mg (45%) von:

Ac-Tyr-Lys-Gly-Trp-Met-Asp-N-methyl-Phe-$NH_2$

ergab.

Dieses Material war bezüglich HPLC Kriterien homogen und ergab die folgende Aminosäureanalyse: Asp 1.00 (1); Gly 1.07 (1); Met 1.00 (1); Tyr 1.12 (1); Lys 1.00 (1); Trp und N-methyl-Phe wurden nicht bestimmt. Es zeigte das korrekte MS.

Summelformel (empirisch bestimmt): $C_{49}H_{62}B_{10}O_{10}S$;
MG: 982.14

Zu 190 mg Pyridinacetylsulfat (PAS) wurden 10 ml trockenes destilliertes Pyridin gegeben. Die daraus resultierende Mischung wurde unter 10-minütigem Rühren auf 60°C erhitzt. Die Lösung wurde abkühlen gelassen und 8 mg in 10 ml Pyridin gelöstes

Ac-Tyr-Lys-Gly-Trp-Met-Asp-N-methyl-Phe

wurde dazugegeben. Die Reaktionsmischung liess man 1 Stunde bei 60°C rühren. Danach wurde die Reaktionsmischung mit 2 Volu mina Ammoniumbicarbonat neutralisiert und lyophilisiert. Die Reinigung wurde durch präparative Umkehrphasen-HPLC an ES Industries $C_{18}$-10 µ Säulen (1,25 × 30 cm) mit einem linearen Gradienten von 10-40% 0,05 M $NH_4HCO_3/CH_3CN$ von 120 Minuten bei einer Flussrate von 5 ml/Min. und Nachweis bei 240 nm durchgeführt, was 7,0 mg (87%)

Ac-Tyr($SO_3H$)-Lys-Gly-Trp-Met-Asp-N-methyl-Phe-$NH_2$

ergab.

Aminosäureanalyse: Asp 1.00(1); Gly 1.00(1); Met 1.00(1); Tyr 1.12(1); Lys 1.00(1); Trp und N-Methyl-Phe wurden nicht bestimmt.

Summenformal (empirisch bestimmt): $C_{49}H_{62}N_{10}O_{13}S_2$;
MG: 1063.21

Beispiel 2

Herstellung von

Tyr($SO_3H$)-Met-Glu-Trp-Met-Thr($SO_3H$)-N-methyl-Phe-$NH_2$

4,0 g (0,8 mMol) Boc-N-methylphenylalanin-Trägermaterial, das, in derselben Weise wie im Beispiel 1 beschrieben, erhalten wurde, wurde gemäss dem Verfahren aus Beispiel 1 einer schrittweisen Festphasensynthese ausgesetzt. Alle Kopplungsschritte wurden wie beschrieben mit den symmetrischen Anhydriden der Boc-Aminosäuren durchgeführt. Bei den Schritten 16 und 20 wurden die aktivierten Aminosäuren mit den entsprechenden Reaktionszeiten zugegeben: 6 individuelle Zyklen wurden mit Boc-O-benzyl-threonin (1.5g, 5 mMol, 60 Min; 1.5g, 5 mMol, 60 Min), Boc-methionin (1.25g, 5 mMol, 30 Min; 1.25 g, 5 mMol, 30 Min); Boc-$N^1$-formyltryptophan (1.7g, 5 mMol, 30 Min; 1.7g, 5 mMol, 30 Min), Boc-glutaminsäure-γ-benzylester (1.6g, 5 mMol, 30 Min.; 1.6g, 5 mMol, 30 Min.), Boc-methionin (1.25g, 5 mMol, 30 Min.; 1.25g, 5 mMol, 30 Min.) und Boc-2, 6-dichlorbenzyltyrosin (2.2g, 5 mMol, 30 Min; 2.2g, 5 mMol, 30 Min) durchgeführt. Abspaltung der Boc-Schutzgruppe wurde wie in Beispiel 1 beschrieben durchgeführt, was 4,9 g des Heptapeptid-Trägermaterials ergab. 1 g des Trägermaterials wurde mit 5 ml HF, der Dimethylsulfid (12 ml), p-Kresol (2,0 ml) und Dithioethan (1 ml) enthielt, 1 Stunde lang bei 0°C gespalten. Nach Einengung auf ein kleines Volumen wurde 18 ml frisch destillierter wasserfreier HF für eine zweite 2-stündige

Umsetzung bei 0°C in das Reaktionsgefäss gegeben. Nach starker Einengung wurde das Trägermaterial mit Ethylacetat gewaschen, dann mit 30%iger Essigsäure behandelt, filtriert und lyophilisiert, was 210 mg des rohen Peptids ergab. 100 mg des rohen Peptids wurden durch präparative HPLC an einer Mikro-Bondapack $C_{18}$ Säule (2,3 × 30 cm) gereinigt. Das Peptid wurde mit einem linearen Gradienten von 5-65% 0,022% TFA/$CH_3CN$ bei einer Flussrate von 8 ml/Min. und Nachweis bei 280 nm eluiert. Der Hauptpeak wurde gesammelt und lyophilisiert, was 15 mg (19%) Tyr-Met-Glu-Trp-Met-Thr-N-methyl-Phe-$NH_2$ ergab. Dieses Material war nach HPLC Kriterien homogen und ergab die richtige Aminosäureanalyse und das korrekte MS.

15 mg (0,02 mMol) des linearen Peptids wurden, wie im einzelnen in Beispiel 1 beschrieben, mit Diphenylphosphorylazid cyclisiert, was 6 mg (41% Ausbeute)

Tyr-Met-Glu-Trp-Met-Thr-N-methyl-Phe-$NH_2$

ergab. Dieses Material war nach HPLC-Kriterien homogen und ergab die folgende Aminosäureanalyse: Thr 0.90(1); Glu 1.00 (1); Met 2.00(2); Tyr 1.00(1); Trp 0.80(1); N-methyl-Phe wurde nicht bestimmt. Es ergab das richtige MS.

Summenformel (empirisch bestimmt): $C_{49}H_{63}N_9O_{10}S_2$;
MG: 1002.0

Zu 80 mg Pyridinacetylsulfat (PAS) wurden 6 ml trockenes destilliertes Pyridin gegeben. Die so erhaltene Mischung wurde unter 10-minütigem Rühren auf 60°C erhitzt. Die Lösung wurde abkühlen gelassen und 3 mg

Tyr-Met-Glu-Trp-Met-Thr-N-

methyl-Phe-$NH_2$ wurden in 3 ml Pyridin gelöst und zur Lösung gegeben. Die Reaktionsmischung wurde 1 Stunde lang bei 60°C gerührt. Danach wurde die Reaktionsmischung mit 2 Volumina Ammoniumbicarbonat neutralisiert und lyophilisiert. Die Reinigung wurde durch präparative HPLC an Umkehrphasen an ES Industries $C_{18}$-10 μ Säulen (1,25 × 30 cm) mit einem linearen Gradienten von 10-40% 0,05 $NH_4HCO_3$/$CH_3CN$ von 120 Minuten bei einer Flussrate von 5 ml/Min. und Nachweis bei 240 nm erreicht, was eine Ausbeute von 1,4 mg (45%)

Tyr($SO_3H$)-Met-Glu-Trp-Met-Thr($SO_3H$)-N-methyl-Phe-$NH_2$

ergab. Aminosäureanalyse: Thr 0.85(1); Glu 1.00(1); Met 1.80(2); Tyr 1.00(1); Trp 0.75(1); N-methyl-Phe wurde nicht bestimmt.

Summenformel (empirisch bestimmt): $C_{49}H_{63}N_9O_{18}S_4$;
MG: 1162.32

Beispiel 3

Herstellung von

Tyr($SO_3H$)-Met-Gly-Trp-Glu-Thr($SO_3H$)-
N-methyl-Phe-$NH_2$

5 g (1 mMol) Boc-N-methylphenylalanin-Trägermaterial, das in der gleichen Weise wie in Beispiel 1 beschrieben erhalten wurde, wurde gemäss demselben wie in Beispiel 1 beschriebenen Verfahren einer

stufenweisen Festphasensynthese ausgesetzt. Alle Kopplungen wurden unter Verwendung der symmetrischen Anhydride der Boc-Aminosäuren wie beschrieben durchgeführt. Bei den Schritten 16 und 20 wurden die aktivierten Aminosäuren mit den entsprechenden Reak tionszeiten zugegeben: 6 individuelle Zyklen wurden mit: Boc-O-benzyl-threonin (1.5 g, 5 mMol, 60 Min; 1,5g, 5 mMol, 60 Min.); Boc-glutaminsäure-γ-benzylester (1.6g, 5 mMol, 30 Min.; 1.6g, 5 mMol, 30 Min.); Boc-N[1]-formyl-tryptophan (1.7g, 5 mMol, 30 Min.; 1.7 g, 5 mMol, 30 Min); Boc-glycin (900 mg, 5 mMol, 30 Min.; 900 mg, 5 mMol, 30 Min.); Boc-methionin (1.25g, 5 mMol, 30 Min.; 1.25g, 5 mMol, 30 Min.) und Boc-2, 6-dichlorbenzyl-tyrosin (2.2g, 5 mMol, 30 Min; 2.2g 5 mMol, 30 Min) durchgeführt.

Die Abspaltung der Boc-Schutzgruppen erfolgt wie in Beispiel 1 beschrieben, was 5,8 g des Heptapeptid-Trägermaterials ergab. 2,4 g des Trägermaterials wurde durch Umsetzung mit Fluorwasserstoff, der Dimethylsulfid, p-Kresol und Dithioethan enthält, unter Verwendung des gleichen wie in Beispiel 2 beschriebenen Verfahrens, gespalten. Das Trägermaterial wurde mit Ethylacetat gewaschen, dann mit 30%iger Essigsäure behandelt, filtriert und lyophilisiert, was 452 mg rohes Peptid ergab.

100 mg des rohen Peptids wurden durch präparative HPLC an einer Mikro-Bondapack-$C_{18}$-Säule (2,3 × 30 cm) gereinigt. Das Peptid wurde mit einem linearen Gradienten von 5-65% 0,022% TFA/$CH_3CN$ bei einer Flussrate von 8 ml/Min. eluiert. Der Nachweise erfolgte bei 280 nm. Die Hauptfraktionen wurden gesammelt und lyophilisiert, was 20 mg (23%) Tyr-Met-Gly-Trp-Glu-Thr-N-methyl-Phe-$NH_2$ ergab. Dieses Material war nach HPLC-Kriterien homogen und gab die korrekte Aminosäureanalyse und das richtige MS.

20 mg (0,02 mMol) des linearen Peptids wurden, wie im einzelnen in Beispiel 1 beschrieben, mit Diphenylphosphorylazid cyclisiert, was 4 mg (21% Ausbeute)

Tyr-Met-Gly-Trp-Glu-Thr-N-methyl-Phe-$NH_2$

ergab.

Dieses Material war bezuglich HPLC-Kriterien homogen und gab die folgende Aminosäureanalyse: Thr 0.90(1); Glu 1.00(1); Gly 1.00(1); Met 0.90(1); Tyr 0.97(1); Trp 0.63(1). N-methyl-Phe wurde nicht bestimmt. Sie ergab das richtige MS.

Summenformel (empirisch bestimmt): $C_{46}H_{57}N_9O_{10}S$;
MG: 928.07

4 mg

Tyr-Met-Gly-Trp-Glu-Thr-N-methyl-Phe-$NH_2$

wurden in 5 ml Pyridin gelöst und zu einer Lösung von 80 mg Pyridinacetylsulfat in 5 ml Pyridin gegeben, die in derselben wie in Beispiel 1 beschriebenen Weise hergestellt worden war. Die Reaktionsmischung wurde 1 Stunde bei 60°C gerührt, anschliessend mit 2 Volumina Ammoniumbicarbonat neutralisiert und lyophilisiert. Die Reinigung wurde durch präparative HPLC unter denselben Bedingungen, wie in Beispiel 1 beschrieben, erreicht. Die Ausbeute betrug 3,8 mg (94%)

Tyr($SO_3H$)-Met-Gly-Trp-Glu-Thr($SO_3H$)-N-methyl-Phe-$NH_2$.

Aminosäureanalyse: Thr 0.90(1); Glu 1.04(1); Gly 1.01(1); Met 0.85(1); N-methyl-Phe und Trp wurden nicht bestimmt.

Summenformel (empirisch bestimmt): $C_{46}H_{57}N_9O_{16}S_3$:
MG 1088.20

Beispiel 4

In vitro Rezeptorbindungsnachweis

Geforenes Rinderstriatum (ungefähr 5 g) und frischer Rattenpankreas (ungefähr 5 g) wurden von Fett und nicht dazugehörendem Gewebe befreit und in HEPES Puffer #1 (10 mM HEPES, 130 mM NaCl, 5 mM $MgCl_2$, pH 7,4) unter Verwendung von 35 Teilen Puffer auf einen Teil gewebe, auf der Grund lage von Nassgewicht/Volumen (ungefähr 175 ml), homogenisiert. Das Gewebe wurde zweimal für ungefähr 15 Sekunden bei 0°C mit einem Homogenisator von Polytron bei einer Einstellung von "6" homogenisiert. Anschliessend wurde bei 48'000 g und 0°C 10 Minuten lang zentrifugiert. Das so erhaltene Gewebspellet wurde in HEPES Puffer #2 (10 mM HEPES, 130 mM NaCl, 5 mM $MgCl_2$, 1 mg/l phenylmethansulfonylfluorid (PMSF), 200 mg/l bacitracin) suspendiert: 1 Teil Striatumgewebe (ursprüngliches Nassgewicht) auf 80 Teile Puffer und 1 Teil Pankreasgewebe (ursprüngliches Nassgewicht) auf 70 Teile Puffer.

Die Inkubation wurde durch Vereinen verschiedener Konzentrationen von nativem CCK-8 oder Peptiden gemäss Formel I mit $^3$H-CCK-8-($SO_3$H) (Endkonzentration 0,15 nM) und Gewebehomogenat (Striatum: 0,26 mg Protein in 2 ml Endvolumen; Pankreas: 0,165 mg Protein in 1 ml Endvolumen) begonnen. Die Proben wurden 30 Minutes bei 25°C inkubiert und die Inkubation durch Giessen der Mischung auf einen vorgefeuchteten GF/B-Filter von Whatman auf einer Mehrfachvacuumabsaugvorrichtung ("Vaçuum Filtration Manifold") von Sandbeck beendet. Die Inkubationsgefässe wurden zweimal mit jeweils 3 ml eiskaltem HEPES-Puffer #2 gewaschen und die Waschflüssigkeit durch einen GF/B-Filter filtriert. Der Filter wurde 10 Minuten lang luftgetrocknet und dann zusammen mit 12 ml "Ready Solv" Zählmischung HP/b von Beckman in ein Zählgefäss gegeben. Die Gefässe wurden 2-12 Stunden lang geschüttelt und deren Inhalt dann in einem Flüssigphasenzählspektrometer Model 7800 von Beckman gezählt. Nicht-spezifische Bindung wurde in Gegenwart von 1 $\mu$M nativem CCK-8 bestimmt und von allen Probenwerten abgezogen, um die spezifische Bindung zu bestimmen. Die Konzentration an Peptid, die nötig war, um 50% der gesamten spezifischen Bindung von $^3$H-CCK-8-($SO_3$H) ($IC_{50}$-Wert) zu inhibieren, wurde durch "log-Probit"-Analyse bestimmt.

Die Ergebnisse sind in Tabelle 1 zusammengefasst.

Tabelle I

| Peptidsequenz | Probe (Beispiel) | Rinder-Striatum (nM) | Ratten-Pankreas (nM) | Dosierung µg/kg | Nahrungsaufnahme 1.Fütterung % Kontrolle | Nahrungsaufnahme 2.Fütterung % Kontrolle |
|---|---|---|---|---|---|---|
| H-Asp-Tyr($SO_3H$)-Met-Gly-Trp-Met-Asp-Phe-$NH_2$ (CCK 8) | | 1-4,6 | 1-3,2 | 32 | 27±17 | 149±6* |
| Ac-Tyr($SO_3H$)-Lys-Gly-Trp-Met-Asp-N-methyl-Phe-$NH_2$ | 1 | 3000 | 1500 | 32 | 72±10 | 133±6* |
| | | | | 320 | 14±5*** | 145±10** |
| Ac-Tyr($SO_3H$)-Lys-Gly-Trp-Met-Asp-Phe-$NH_2$ | | >1000 | 45 | 32 | 81±6* | 111±4 N.S. |
| | | | | 320 | 43±7*** | 135±5*** |
| Ac-Tyr($SO_3H$)-Lys-Gly-Trp-Nle-Asp-N-methyl-Phe-$NH_2$ | | >1000 | 6600 | 320 | 77±10* | 132±10** |
| Ac-Tyr($SO_3H$)-Orn-Gly-Trp-Met-Asp-N-methyl-Phe-$NH_2$ | | 52000 | 13000 | 320 | 72±10* | 141±9* |
| Tyr($SO_3H$)-Met-Glu-Trp-Met-Thr($SO_3H$)-N-methyl-Phe-$NH_2$ | 2 | >1000 | 5400 | 320 | 52±7*** | 131±9** |
| Tyr($SO_3H$)-Met-Gly-Trp-Glu-Thr($SO_3H$)-N-methyl-Phe-$NH_2$ | 3 | >10000 | >10000 | 320 | 73±10* | 114±13 N.S. |

Signifikanz der Werte bezüglich der Kontrollen:

*** $p \leq 0.001$, ** $p \leq 0.01$, * $p \leq 0.05$

N.S. = nicht signifikant

Beispiel 5

Fütterungstest

Männliche Sprague-Dawley (CD) Ratten mit einem Gewicht von 180-200 g (Charles River Breeding Laboratories) wurden an einen 12-stündigen Hell/Dunkel-Zyklus von 6 Uhr morgens bis 6 Uhr abends in einem auf 22°C temperierten Raum gewöhnt. Anschliessend liess man sie 2 Tage lang fasten, sie wurden gewogen, in individuelle Käfige gesetzt und einem 4 Tage lang dauernden Fütterungstraining unterzogen. Während dieser Zeit gab man den Ratten 1 Stunde lang, d.h. von 9 Uhr bis 10 Uhr morgens Laborgrundnahrung (Purin Lab Chow) in die Futtertröge, die von 10 bis 12 Uhr entfernt und von 12 bis 1 Uhr in die Käfige zurückgegeben wurden. Durch dieses 1-2-1 Verfütternungssystem lernen die meisten Ratten während der 2 Stunden pro Tag, in denen sie Zugang zum Futter haben annähernd so viel zu fressen, wie Ratten, die 24 Stunden lang beliebig viel Nahrung zur Verfügung haben. Am 4. Tag wurden die Ratten nochmals gewogen und jede, die mahr als 5 g pro Körpergewicht verloren hatte, wurde vom Test ausgeschlossen. Die Tiere wurden dann in eine Versuchs-(n = 5-6) und eine Kontrollgruppe (n = 6-12) aufgeteilt, wobei Tiere gleichen Körpergewichts nicht zusammengefasst wurden.

Erfindungsgemässe Peptide wurden in Konzentrationen von 32-320 µg/kg Körpergewicht, falls löslich in Kochsalzlösung oder falls unlöslich in 1% Gummi arabicum, suspendiert und am 5. Tag des Tetes 15 Minuten vor der ersten Fütterung intraperitioneal appliziert. Den Ratten wurde ihr Fressen dann genau wie an den vorhergehenden 4 Tagen gegeben. Die Fressnäpfe wurden vor und nach der Fütterung gewogen, um die Menge der Nahrungsaufnahme zu bestimmen. Werte für die Menge an aufgenommener Nahrung wurden als Mittelwerte und Standardabweichungen von Mittelwerten als Prozentsatz der Kontrollwerte für die verschiedenen Gruppen ausgedrückt. Die Werte der Versuchsgruppen wurden durch t-Testanalyse mit denen der Kontrollgruppen verglichen. Die Ergebnisse sind in Tabelle I zusammengefasst.

Beispiel 6

400 µl Magensaft und 200 µg des Peptids aus Beispiel 1

[Ac-Tyr($SO_3H$)-Lys-Gly-Trp-Met-Asp-N-methyl-Phe-$NH_2$]

bzw. CCK-8 [H-Asp-Tyr($SO_3H$)-Met-Gly-Trp-Met-Asp-Phe-$NH_2$] wurden mit verschiedenen Zeiten bei 37°C inkubiert. Das Ausmass des Abbaus wurde durch analytische HPLC auf einer Mikro-Bondapack-$C_{18}$-Säule (0,4 × 30 cm) mit einem linearen Gradienten von 10-40% 0,01 M $NH_4Ac/CH_3CN$ von 40 Minuten bei einer Flussrate von 2 ml/Min. und Nachweis bei 225 nm verfolgt.

Abbauraten für Beispiel 1 beruhen auf einer angenommenen Produktion an unsulfatiertem

Ac-Tyr-Lys-Gly-Trp-Met-Asp-N-

methyl-Phe-$NH_2$ und für CCK-8 auf dem Verschwinden des CCK-8-Chromatogramgipfels.

| Peptid | Inkubationszeit (Min.) | Abbau (Flächen-%) |
|---|---|---|
| Beispiel 1 | 0 | 0 |
| | 60 | 1.4 |
| | 180 | 3.6 |
| | 1380 | 12.8 |
| CCK-8 | 0 | 0 |
| | 15 | 90 |
| | 60 | 100 |

**Ansprüche**

1. Ein Peptid der Formel

$$X\text{-}Tyr(SO_3H)\text{-}R\text{-}Gly\text{-}Trp\text{-}R^1\text{-}Asp\text{-}R^2\text{-}Y \qquad I$$

worin
X = CO-niederes Alkyl
R = Lys, Orn
$R^1$ = Met, Nle
$R^2$ = Phe, N-methyl-Phe
Y = $NH_2$, $NHCH_3$
und dessen pharmazeutisch sinnvolle Salze.

2. Das Peptid gemäss Anspruch 1, worin X = $CO\text{-}CH_3$, R = Lys, Orn; $R^1$ = Met, Nle; $R^2$ -N-methyl-Phe oder Phe; und Y = $NH_2$.

3. Das Peptid gemäss Anspruch 2, worin R = Lys; $R^1$ = Met, Nle; $R^2$ = Phe, N-methyl-Phe.

4. Das Peptid gemäss Anspruch 3, worin $R^1$ = Met; und $R^2$ = N-methyl-Phe mit der Formel

$$CH_3\text{-}CO\text{-}Tyr(SO_3H)\text{-}Lys\text{-}Gly\text{-}Trp\text{-}Met\text{-}Asp\text{-}N\text{-}methyl\text{-}Phe\text{-}NH_2$$

5. Das Peptid gemäss Anspruch 3, worin $R^1$ = Met; $R^2$ = Phe mit der Formel

$$CH_3\text{-}CO\text{-}Tyr(SO_3H)\text{-}Lys\text{-}Gly\text{-}Trp\text{-}Met\text{-}Asp\text{-}Phe\text{-}NH_2$$

6. Das Peptid gemäss Anspruch 3, worin $R^1$ = Nle; $R^2$ = N-methyl-Phe mit der Formel

$$CH_3\text{-}CO\text{-}Tyr(SO_3H)\text{-}Lys\text{-}Gly\text{-}Trp\text{-}Nle\text{-}Asp\text{-}N\text{-}methyl\text{-}Phe\text{-}NH_2$$

7. Das Peptid gemäss Anspruch 2, worin R = Orn und $R^1$ = Met; $R^2$ = N-methyl-Phe mit der Formel

$CH_3$-CO-Tyr($SO_3$H)-Orn-Gly-Trp-Met-Asp-N-methyl-Phe-$NH_2$

8. Ein Peptid der Formel

Tyr($SO_3$H)-R-Glu-Trp-$R^1$-$R^2$-N-methyl-Phe-Y II

worin
R = Met, Nle
$R^1$ = Met, Nle
$R^2$ = Thr($SO_3$H), Ser($SO_3$H), Hyp($SO_3$H)
Y = $NH_2$, $NHCH_3$
und dessen pharmazeutisch sinnvolle Salze.

9. Das Peptid gemäss Anspruch 8, worin R= Met, Nle; $R^1$ = Met, Nle; $R^2$ = Thr($SO_3$H), Ser($SO_3$H), Hyp($SO_3$H); und Y = $NH_2$.

10. Das Peptid gemäss Anspruch 9, worin R = Met; $R^1$ = Met, Nle; $R^2$ = Thr($SO_3$H), Ser ($SO_3$H), Hyp-($SO_3$H).

11. Das Peptid gemäss Anspruch 10, worin $R^1$ = Met und $R^2$ = Thr($SO_3$H) mit der Formel

Tyr($SO_3$H)-Met-Glu-Trp-Met-Thr($SO_3$H)-N-methyl-Phe-$NH_2$

12. Ein Peptid der Formel

Tyr($SO_3$H)-R-Gly-Trp-Glu-$R^1$-N-methyl-Phe-Y III

worin
R = Met, Nle
$R^1$ = Thr($SO_3$H), Ser($SO_3$H), Hyp($SO_3$H)
Y = $NH_2$, $NHCH_3$
und dessen pharmazeutisch sinnvolle Salze.

13. Das Peptid gemäss Anspruch 12, worin R = Met, Nle; $R^1$ = Thr($SO_3$H), Ser($SO_3$H), Hyp($SO_3$H) und Y = $NH_2$.

14. Das Peptid gemäss Anspruch 13, worin R = Met; $R^1$ = Thr($SO_3$H), Ser($SO_3$H), Hyp($SO_3$H).

15. Das Peptid gemäss Anspruch 14, worin $R^1$ = Thr($SO_3$H) mit der Formel

Tyr($SO_3$H)-Met-Gly-Trp-Glu-Thr($SO_3$H)-N-methyl-Phe-$NH_2$

16. Ein Peptid der Formel

X-Tyr($SO_3$H)-R-Gly-Trp-$R^1$-Asp-$R^2$-Y IV

worin
X = CO-niederes Alkyl
R = Lys, Orn
$R^1$= Met, Nle

$R^2$ = Phe, N-methyl-Phe
Y = $NH_2$, $NHCH_3$
und dessen pharmazeutisch sinnvolle Salze.

17. Ein Peptid der Formel

Tyr($SO_3H$)-R-Glu-Trp-$R^1$-$R^2$-N-methyl-Phe-Y V

worin
R = Met, Nle
$R^1$ = Met, Nle
$R^2$ = Thr($SO_3H$), Ser($SO_3H$), Hyp($SO_3H$)
Y = $NH_2$, $NHCH_3$
und deren pharmazeutisch sinnvolle Salze.

18. Ein Peptid der Formel

Tyr($SO_3H$)-R-Gly-Trp-Glu-$R^1$-N-methyl-Phe-Y VI

worin
R = Met, Nle
$R^1$ = Thr($SO_3H$), Ser($SO_3H$), Hyp($SO_3H$)
Y = $NH_2$, $NHCH_3$
und dessen pharmazeutisch sinnvolle Salze.

19. Peptide oder deren pharmazeutisch sinnvolle Salze gemäss wenigstens einem der Ansprüche 1-18 zur Verwendung als therapeutisch wirksame Substanzen.

20. Peptide oder deren pharmazeutisch sinnvolle Salze gemäss wenigstens einem der Ansprüche 1-18 zur Verwendung als Substanzen zur Kontrolle oder Unterdrückung der Nahrungsaufnahme.

21. Ein Verfahren zur Herstellung der Peptide oder deren pharmazeutisch sinnvoller Salze gemäss wenigstens einem der Ansprüche 1-18, dadurch gekennzeichnet, dass man die entsprechenden unsulfatierten Peptide mit einem Sulfatierungsreagenz umsetzt und, falls gewünscht, ein so erhaltenes Peptid in ein pharmazeutisch sinnvolles Salz überführt.

22. Ein pharmazeutisches Präparat, das ein Peptid oder ein Pharmazeutisch sinnvolles Salz davon gemäss wenigstens einem der Ansprüche 1-18 und ein nicht-toxisches, inertes, therapeutisch verträgliches Trägermaterial enthält.

23. Ein pharmazeutisches Präparat gemäss Anspruch 22 zur Kontrolle oder Unterdrückung der Nahrungsaufnahme.

24. Die Verwendung eines Peptids oder eines pharmazeutisch sinnvollen Salzes davon gemäss wenigstens einem der Ansprüche 1-18 bei der Kontrolle oder Verhinderung von Krankheiten.

25. Die Verwendung eines Peptids oder eines pharmazeutisch sinnvollen Salzes davon gemäss wenigstens einem der Ansprüche 1-18 in der Kontrolle oder Unterdrückung von Nahrungsaufnahme.

<u>Patentansprüche für folgende Vertragsstaaten: GR und ES</u>

1. Ein Verfahren zur Herstellung eines Peptids der Formel

$$X\text{-}Tyr(SO_3H)\text{-}\underline{R\text{-}Gly\text{-}Trp\text{-}R^1\text{-}Asp}\text{-}R^2\text{-}Y \qquad I$$

worin
X = CO-Niederes Alkyl
R = Lys, Orn
$R^1$ = Met, Nle
$R^2$ = Phe, N-methyl-Phe
Y = $NH_2$, $NHCH_3$
und dessen pharmazeutisch sinnvoller Salze, dadurch gekennzeichnet, dass man das entsprechende unsulfatierte Peptid mit einem Sulfatierungsreagenz umsetzt und, falls erwünscht, das so erhaltene Peptid in ein pharmazeutisch sinnvolles Salz überführt.

2. Ein Verfahren zur Herstellung des Peptids gemäss Anspruch 1, worin X = CO-$CH_3$, R = Lys, Orn; $R^1$ = Met, Nle; $R^2$ = N-methyl-Phe oder Phe; und Y = $NH_2$.

3. Ein Verahren zur Herstellung des Peptids gemäss Anspruch 2, worin R = Lys; $R^1$ = Met, Nle; $R^2$ - Phe, N-methyl-Phe.

4. Ein Verfahren zur Herstellung des Peptids gemäss Anspruch 3, worin $R^1$ = Met; und $R^2$ = N-methyl-Phe mit der Formel

$CH_3$-CO-Tyr($SO_3$H)-Lys-Gly-Trp-Met-Asp-N-methyl-Phe-$NH_2$

5. Ein Verfahren zur Herstellung des Peptids gemäss Anspruch 3, worin $R^1$ = Met; $R^2$ = Phe mit der Formel

$CH_3$-CO-Tyr($SO_3$H)-Lys-Gly-Trp-Met-Asp-Phe-$NH_2$

6. Ein Verfahren zur Herstellung des Peptids gemäss Anspruch 3, worin $R^1$ = Nle; $R^2$ = N-methyl-Phe mit der Formel

$CH_3$-CO-Tyr($SO_3$H)-Lys-Gly-Trp-Nle-Asp-N-methyl-Phe-$NH_2$

7. Ein Verfahren zur Herstellung des Peptids gemäss Anspruch 2, worin R = Orn und $R^1$ = Met; $R^2$ = N-methyl-Phe mit der Formel

$CH_3$-CO-Tyr($SO_3$H)-Orn-Gly-Trp-Met-Asp-N-methyl-Phe-$NH_2$

8. Ein Verfahren zur Herstellung eines Peptids der Formel

Tyr($SO_3$H)-R-Glu-Trp-$R^1$-$R^2$-N-methyl-Phe-Y     II

worin
R = Met, Nle
$R^1$ = Met, Nle
$R^2$ = Thr($SO_3$H), Ser($SO_3$H), Hyp($SO_3$H)
Y = $NH_2$, $NHCH_3$
und dessen pharmazeutisch sinnvoller Salze, dadurch gekennzeichnet, dass man das entsprechende unsulfatierte Peptid mit einem Sulfatierungsreagenz umsetzt und falls erwünscht, das so erhaltene Peptid in ein pharmazeutisch sinnvolles Salz überführt.

9. Ein Verfahren zur Herstellung des Peptids gemäss Anspruch 8, worin R = Met, Nle; $R^1$ = Met, Nle; $R^2$ = Thr($SO_3$H), Ser($SO_3$H), Hyp($SO_3$H); und Y = $NH_2$.

10. Ein Verfahren zur Herstellung des Peptids gemäss Anspruch 9, worin R = Met; $R^1$ = Met, Nle; $R^2$ = Thr($SO_3$H), Ser($SO_3$H), Hyp($SO_3$H).

11. Ein Verfahren zur Herstellung des Peptids gemäss Anspruch 10, worin $R^1$ = Met und $R^2$ = Thr-($SO_3$H) mit der Formel

Tyr($SO_3$H)-Met-Glu-Trp-Met-Thr($SO_3$H)-N-methyl-Phe-$NH_2$

12. Ein Verfahren zur Herstellung eines Peptids der Formel

Tyr($SO_3H$)-R-Gly-Trp-Glu-$R^1$-N-methyl-Phe-Y    III

worin
R = Met, Nle
$R^1$ = Thr($SO_3H$), Ser($SO_3H$), Hyp($SO_3H$)
Y = $NH_2$, $NHCH_3$
und dessen pharmazeutisch sinnvoller Salze, dadurch gekennzeichnet, dass man das entsprechende unsulfatierte Peptid mit einem Sulfatierungsreagenz umsetzt und, falls erwünscht, das so erhaltene Peptid in ein pharmazeutisch sinnvolles Salz überführt.

13. Ein Verfahren zur Herstellung des Peptids gemäss Anspruch 12, worin R = Met, Nle; $R^1$ = Thr($SO_3H$), Ser($SO_3H$), Hyp($SO_3H$) und Y = $NH_2$.

14. Ein Verfahren zur Herstellung des Peptids gemäss Anspruch 13, worin R = Met; $R^1$ = Thr($SO_3H$), Ser($SO_3H$), Hyp($SO_3H$).

15. Ein Verfahren zur Herstellung des Peptids gemäss Anspruch 14, worin $R^1$ = Thr($SO_3H$) mit der Formel

Tyr($SO_3H$)-Met-Gly-Trp-Glu-Thr($SO_3H$)-N-methyl-Phe-$NH_2$

16. Ein Verfahren zur Herstellung eines Peptids der Formel

X-Tyr($SO_3H$)-R-Gly-Trp-$R^1$-Asp-$R^2$-Y    IV

worin
X = CO-niederes Alkyl
R = Lys, Orn
$R^1$ = Met, Nle
$R^2$ = Phe, N-methyl-Phe
Y = $NH_2$, $NHCH_3$
und dessen pharmazeutisch sinnvoller Salze, dadurch gekennzeichnet, dass man das entsprechende unsulfatierte Peptid mit einem Sulfatierungsreagenz umsetzt und, falls erwünscht, das so erhaltene Peptid in ein pharmazeutisch sinnvolles Salz überführt.

17. Ein Verfahren zur Herstellung eines Peptids der Formel

Tyr($SO_3H$)-R-Glu-Trp-$R^1$-$R^2$-N-methyl-Phe-Y    V

worin
R = Met, Nle
$R^1$ = Met, Nle
$R^2$ = Thr($SO_3H$), Ser ($SO_3H$), Hyp($SO_3H$)
Y = $NH_2$, $NHCH_3$
und dessen pharmazeutisch sinnvoller Salze, dadurch gekennzeichnet, dass man das entsprechende unsulfatierte Peptid mit einem Sulfatierungsreagenz umsetzt und, falls erwünscht, das so erhaltene Peptid in ein pharmazeutisch sinnvolles Salz überführt.

18. Ein Verfahren zur Herstellung eines Peptids der Formel

Tyr($SO_3H$)-R-Gly-Trp-Glu-$R^1$-N-methyl-Phe-Y    VI

worin
R = Met, Nle
$R^1$ = Thr($SO_3H$), Ser($SO_3H$), Hyp($SO_3H$)
Y = $NH_2$, $NHCH_3$

und dessen pharmazeutisch sinnvoller Salze, dadurch gekennzeichnet, dass man das entsprechende unsulfatierte Peptid mit einem Sulfatierungsreagenz umsetzt und, falls erwünscht, das so erhaltene Peptid in ein pharmazeutisch sinnvolles Salz überführt.

19. Ein Verfahren zur Herstellung eines pharmazeutischen Präparates, insbesondere eines solchen zur Ueberwachung oder Unterdrückung der Nahrungsaufnahme, dadurch gekennzeichnet, dass man ein Peptid gemäss mindestens einem der Ansprüche 1-18 oder ein pharmazeutisch sinnvolles Salz davon und, falls gewünscht, eine oder mehrere andere therapeutisch wirksame Substanzen mit einem therapeutisch inerten Trägermaterial vermischt und die Mischung in eine galenische Applikationsform bringt.

20. Die Verwendung eines Peptids gemäss mindestens einem der Ansprüche von 1-18 oder eines pharmazeutisch sinnvollen Salzes davon zur Herstellung eines pharmazeutischen Präparates zur Ueberwachung oder Verhinderung von Krankheiten.

21. Die Verwendung eines Peptids gemäss mindestens einem der Ansprüche von 1-18 oder eines pharmazeutisch sinnvollen Salzes davon zur Herstellung eines pharmazeutischen Präparates zur Ueberwachung oder Unterdrückung der Nahrungsaufnahme.

22. Ein Peptid der Gruppe bestehend aus Verbindungen der Formeln:

X-Tyr($SO_3H$)-R-Gly-Trp-$R^1$-Asp-$R^2$-Y IV

worin
X = CO-niederes Alkyl
R = Lys, Orn
$R^1$ = Met, Nle
$R^2$ = Phe, N-methyl-Phe
Y = $NH_2$, $NHCH_3$

Tyr($SO_3H$)-R-Glu-Trp-$R^1$-$R^2$-N-methyl-Phe-Y V

worin
R = Met, Nle
$R^1$ = Met, Nle
$R^2$ = Thr($SO_3H$), Ser($SO_3H$), Hyp($SO_3H$)
Y = $NH_2$, $NHCH_3$

Tyr($SO_3H$)-R-Gly-Trp-Glu-$R^1$-N-methyl-Phe-Y VI

worin
R = Met, Nle
$R^1$ = Thr($SO_3H$), Ser($SO_3H$), Hyp($SO_3H$)
Y = $NH_2$, $NHCH_3$.